**Europäisches Patentamt**

**European Patent Office**

**Office européen des brevets**

(19)

(11) Veröffentlichungsnummer : **0 445 385 B1**

(12) # EUROPÄISCHE PATENTSCHRIFT

(45) Veröffentlichungstag der Patentschrift :
**18.08.93 Patentblatt 93/33**

(51) Int. Cl.⁵ : **C12N 15/74, C12N 15/77, C12N 15/70**

(21) Anmeldenummer : **90123263.7**

(22) Anmeldetag : **05.12.90**

(54) **Transposon, dieses Transposon enthaltende Testvektoren und Verfahren zur Mutagenese.**

(30) Priorität : **03.03.90 DE 4006637**

(43) Veröffentlichungstag der Anmeldung :
**11.09.91 Patentblatt 91/37**

(45) Bekanntmachung des Hinweises auf die
Patenterteilung :
**18.08.93 Patentblatt 93/33**

(84) Benannte Vertragsstaaten :
**AT BE CH DE DK ES FR GB IT LI NL SE**

(56) Entgegenhaltungen :
**Patent Abstracts of Japan, Band 10, Nr. 43 (C-329)(2100), 20. Februar 1986**

(73) Patentinhaber : **Degussa Aktiengesellschaft Weissfrauenstrasse 9 D-60311 Frankfurt (DE)**

(72) Erfinder : **Kassing, Friedrich Körnerstrasse 4 W-4830 Gütersloh 1 (DE)**
Erfinder : **Schäfer, Andreas Bünderstrasse 33 W-4800 Bielefeld 1 (DE)**
Erfinder : **Kalinowski, Jörn, Dr. Schlosshofstr. 123 W-4800 Bielefeld 1 (DE)**
Erfinder : **Pühler, Alfred, Prof. Dr. Am Waldschlöschen 2 W-4800 Bielefeld 15 (DE)**

EP 0 445 385 B1

## Beschreibung

Die Erfindung betrifft ein Transposon, bestehend aus einem Fragment des R-Plasmids pCxM82B, dieses Transposon enthaltende Testvektoren und Verfahren zur Mutagenese.

Ein Transposon enthält eine DNA-Sequenz, die für ein Protein kodiert, das es diesem Element ermöglicht, durch illegitime Rekombination in die DNA eines Chromosoms bzw. eines Plasmids unspezifisch zu integrieren. Weiterhin besitzt ein Transposon eine Selektionsmarkierung (z. B. ein Antibiotika-Resistenzgen), mit deren Hilfe man auf die Anwesenheit eines Transposons in einer Zelle selektionieren kann.

Dies ermöglicht die direkte Identifizierung von Integrationsmutanten und die Lokalisierung des betroffenen Gens im Genom. Die Isolierung eines Restriktionsfragments mit dem betroffenen Gen und dem inserierten Transposon kann dann nach dem Stand der Technik erfolgen.

Mit herkömmlichen Mutagenese-Verfahren (Hydroxylamin-Mutagenese bzw. NTG-Mutagenese) wird dagegen oft die Vitalität der Organismen negativ beeinflußt. Die schnelle Lokalisierung des mutierten Gens stellt bei diesem Verfahren ebenfalls ein großes Problem dar.

Die Technik der Transposon-Mutagenese wird bei Gram-positiven und Gram-negativen Bakterien bereits erfolgreich eingesetzt. Beispielhaft sollen hier die Transposons Tn10 (9,3 Kb, Tc$^r$) vom Plasmid R100 (Kleckner et al., 1981), Tn5 (5,7 kB, Km$^r$) vom Plasmid JR67 (Berg et al., 1975) aus Gram-negativen und die Transposons Tn917 (5,1 Kb, Em$^r$) vom Plasmid pAD2 (Tomich et al., 1980) und Tn916 (15 Kb, Tc$^r$) aus dem Chromosom von Streptococcus faecalis DS16 aus Gram-positiven Bakterien erwähnt werden.

Das Transposon Tn917 wurde in Bacillus ssp sowie in anderen Gram-positiven und Gram-negativen Organismen (Kuramitsu and Casadaban, 1986) erfolgreich zur Erzeugung von auxotrophen Zellen sowohl des Aminosäurestoffwechsels als auch des Energiestoffwechsels eingesetzt (Perkins and Youngman, 1984; Vandeyar and Zahler, 1986, Mc Laughlin dand Hughes, 1989).

Weiterhin werden Transposons zur Genomkartierung, zur Mobilisierung von Replikons, zur Erzeugung von Operon-Fusionen und zur Induktion von Genen (Simon et al., 1989) benutzt.

Die bekannten Transposon-Mutagenesesysteme eignen sich jedoch nicht für die gentechnische Veränderung von coryneformen Bakterien, insbesondere solchen, die Aminosäuren produzieren und ausscheiden.

Aufgabe der Erfindung ist es, ein derartiges Transposon-Mutagenesesystem zur Verfügung zu stellen, das neue Möglichkeiten in der genetischen Analyse insbesondere von coryneformen Bakterien bietet.

Es wurde nun gefunden, daß ein bestimmtes Fragment des R-Plasmids pCxM82b Transposonaktivität zeigt.

Das R-Plasmid ist bei der Deutschen Sammlung von Mikroorganismen nach dem Budapester Vertrag in Corynebakterium xerosis DSM 5021 hinterlegt und in der europäischen Patentanmeldung No. 89 120 459.6 (entspr. der deutschen Patentanmeldung P 38 41 454.6) beschrieben.

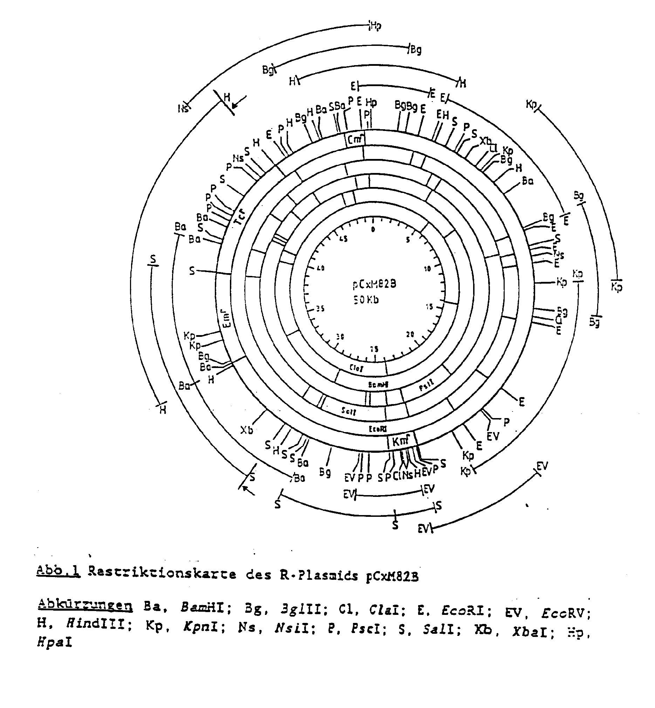

Die zirkuläre Karte ist in Abb. 1 enthalten.

Dieser sind auch die weiteren Kennzeichen des beanspruchten Fragments zu entnehmen:

a) der für die Integration essentielle DNA-Bereich liegt zwischen den Koordinaten 30,4 - 44,8 Kb auf dem R-Plasmid,

b) das Fragment wird an der Koordinate 30,4 Kb durch eine SalI-Schnittstelle und an der Koordinate 44,8 Kb durch eine HindIII-Schnittstelle begrenzt, und

c) enthält für eine Tetracyclin- und Erythromycinresistenz codierende DNA-Abschnitte.

Die Transposonaktivität zeigt sich sowohl in Gram-positiven Bakterien, insbesondere in coryneformen Bakterien, wie z.B. Corynebacterium (C.) glutamicum, C. xerosis, C. acetoacidophilum, C. herculis, C. lilium, C. melassecola Brevibacterium (B.) flavum, B. thiogenitalis, B. lactofermentum, B. roseum, B. saccharoliticum, insbesondere Brevibacterium lactofermentum DSM 20412, Corynebacterium callunae DSM 20147, Corynebacterium herculis DSM 20301, Brevibacterium flavum DSM 20411, Arthrobacter albidus DSM 20128, wobei solche bevorzugt werden, die Aminosäuren produzieren und ausscheiden.

Das Transposon wird mit Hilfe eines geeigneten Trägerplasmids entweder durch Transformation oder durch Konjugation in den Rezipienten eingeführt.

Dieses Trägerplasmid kann im Rezipienten nicht oder nur bei niedrigen Inkubationstemperaturen replizieren (temperatursensitive Replikation)

Besonders geeignet sind mobilisierbare, nicht selbsttransferierbare Vektoren (Testvektoren) die zusammengesetzt sind aus

a) einem DNA-Segment, enthaltend ein in E.coli funktionelles Replikon,

b) einem zweiten DNA-Segment, enthaltend die für die Mobilisierungs- und Transferfunktion codierenden DNA-Fragmente (Mob-site und ori T) und

c) das Transposon

Zur Konstuktion derartiger Testvektoren werden mobilisierbare nicht selbsttransferierbare E.coli-Vektoren verwendet.

Der Begriff E.coli-Vektoren beinhaltet allgemein alle nur in E.coli-Stämmen selbständig replizierenden Plasmide, die sich nach dem Stand der Technik als für gentechnologische Anwendungen nützlich erwiesen haben.

Beispiel solcher E.coli-Vektoren sind pMB9, pBR322, pBR325, pKB111, pUC8, pUC9, pACYC184, pACYC177, pSC101.

Diese und anderen Vektoren, die nur in Bakterienstämmen der E.coli-Gruppe replizieren, werden durch Insertion der Mob-site eines Plasmides mit weitem Wirtsbereich in Gram-negativen Bakterien modifiziert.

Bevorzugt wird das Plasmid RP4 für diese Zwecke verwendet. E.coli-Vektoren,d ie ein 1,9 Kb großes Fragment (Mob-site) von RP4 tragen, lassen sich in dem erfindungsgemäßen Verfahren vorteilhaft einsetzen.

Als Mobilisatorstämme geeignet sind modifizierte E.coli-Stämme, die ein Plasmid im Chromosom integriert oder frei vorliegend enthalten, das in der Lage ist, die zur Mobilisierung notwendigen Funktionen bereitzustellen.

Es eignen sich insbesondere Stämme, in deren Chromosom ein RP4-Derivat integriert ist, dessen Transfer-Funktion in trans auf die Mob-site der oben genannten Vektoren einwirkt.

Geeignete Vektoren und E.coli Mobilisatorstämme sind aus der US-PS 4,626,504 bekannt und beim Nothern Regional Research Center hinterlegt:

| Stämme | NRRL-Hinterlegungsnummer |
|---|---|
| E.coli CSH52/pSUP101 | B - 15484 |
| E.coli CSH52/pSUP201 | B - 15487 |
| E.coli CSH52/pSUP202 | B - 15488 |
| E coli CSH52/pSUP203 | B - 15489 |
| E.coli CSH52/pSUP301 | B - 15492 |
| E.coli CSH52/pSUP401 | B - 15494 |
| E.coli SM10 | B - 15481 |
| E.coli S68-7 | B - 15482 |
| E.coli S17-1 | B - 15483 |

Tabelle 1

Weitere Vektoren, wie pSUP102 oder pSUp205 sind aus der Literatur bekannt und werden nach analogen Verfahren aus bekannten Vektoren gewonnen (Simon et al., Methods of Enzymology 118, 640 ff (1986) und Biotechnology, November 1982)1

Die unter Verwendung der genannten E.coli-Vektoren konstruierten, das Transposon enthaltenden Testvektoren werden durch Konjugation aus E.coli Mobilisatorstämmen in die Gram-positiven Bakterien gemäß einem Verfahren transferiert, wie es in der europäischen Patentanmeldung No. 89 120 473.7 (entspr. der deutschen Patentanmeldung P 38 41 453.8) beschrieben wird.

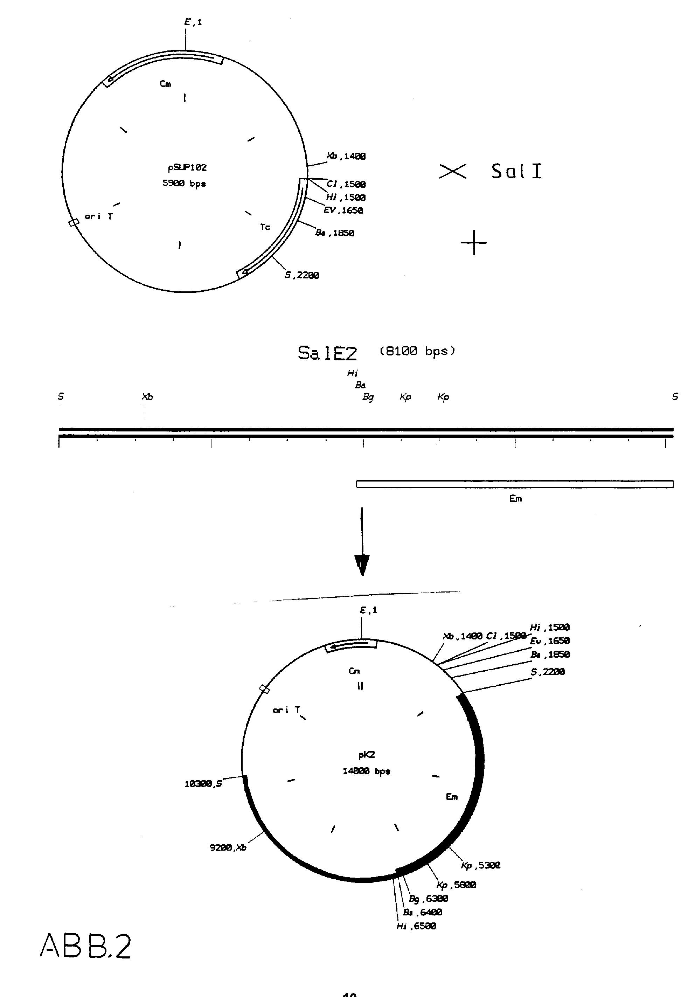

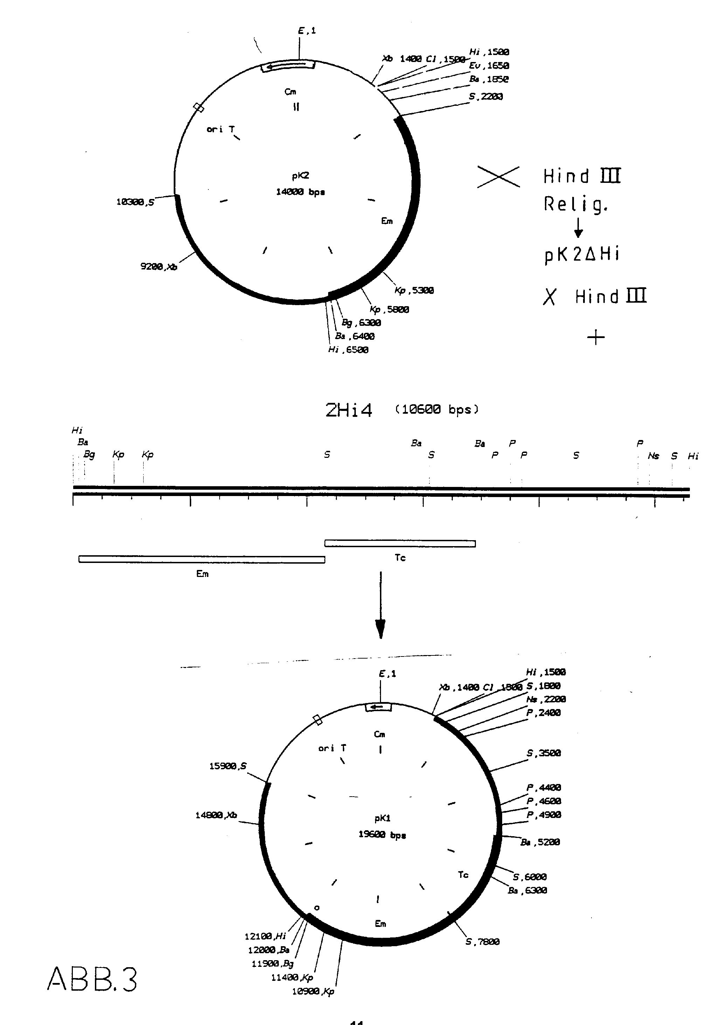

Der erfindungsgemäß konstruierte Testvektor pK1 (s. Abb. 2 und 3) besteht aus dem Transposon und einem durch eine HindIII- und eine SalI-Schnittstelle begrenzten Fragment des Vektors pSUP102 und hat eine Gesamtgröße von ~19,7 Kb.

Dabei stellt man restriktionsdefekte Zelles eines Gram-positiven Bakteriums her und mischt diese nach an sich bekannten Kreuzungsverfahren mit einem den mobilisierbaren Testvektor tragenden E.coli Mobilisatorstamm.

Während sich der Donor bevorzugt in der logarithmischen Wachstumsspase befindet, hat sich für den Zustand des Rezipienten die stationäre Wachstumsphase als günstig erwiesen.

Donor- und Rezipientenzellen werden im allgemeinen im Verhältnis 1 : 1 bis 1 : 10, bevorzugt 1 : 1 bis 1 : 6 eingesetzt.

Der Restriktionsdefekt kann genetisch bedingt sein und z. B. durch mutagene Agentien (z. B. NTG:Methylnitronitrosoguanidin), erzeugt werden, er kann aber auch physiologisch bedingt sein,z. B. durch einen Hitzeschock.

Als besonders effektiv hat sich die Hitzebehandlung des Rezipienten unmittelbar vor der Kreuzung erwiesen.

Dabei sind intakte oder sphäroplastierte Zellen einzusetzen.

Ein Hitzeschock für die Dauer von 1 bis 30 min, bevorzugt ca. 9 min, bei 45 bis 55 °C, bevorzugt ca. 49 °C, ermöglicht es, mit der sich daran anschließenden Steigerung der Transferfrequenz die Aufgabe der Erfindung zu erfüllen.

Die Konstuktion mobilisierbarer Vektoren wird ebenfalls in der europäischen Patentanmeldung No. 89 130 473.7 ≙ EP-A-0375 889 beschrieben.

Zur Prüfung der Transposonaktivität inseriert man das ~14,4 Kb-Fragment des R-Plasmids in einen mobilisierbaren Vektor und führt dieses Konstrukt via Konjugation in ein coryneformes Bakterium, insbesondere ein Aminosäuren produzierendes, ein.

Bevorzugt wählt man ein Corynebakterium glutamicum aus.

Der in diesem Bakterium nicht replizierende erfindungsgemäße Testvektor geht in der Rezipientenzelle größtenteils verloren und mit ihm das inserierte DNA-Fragment des R-Plasmids.

Zum ersten Mal wurde aber nun gefunden, daß ein Transposon-Testvektor in das Chromosom eines als Rezipienten dienenden coryneformen Bakteriums in merklichen Umfang integriert bzw., daß es zur Integration eines bestimmtenDNA-Segments der R-Plasmid-DNA in das Chromosom des Rezipienten kommt (Transposition).

Die Integrationen erfolgen an unterschiedlichen Stellen des Chromosoms.

Eine Untersuchung der unter Verwendung des Testvektors pK1 (Abb. 3) erhaltenen Integrationsmutanten zeigt, daß der Vektor pK1 nach Mobilisierung von E.coli nach C.glutamicum unspezifisch in das Chromosom des als Rezipienten gewählten Stammes C.glutamicum ATCC13032 integriert.

Bei den durchgeführten Transposon-Mutagenese Ansätzen wurden C.glutamicum-Zellen mit drei unterschiedlichen Auxotrophien gefunden (Al Leu⁻, AZ Trp⁻, A3 Ser⁻).

Voraussetzung für derartige Ereignisse ist das Vorhandensein eines Transposons bzw. eines Insertionselements auf dem Testvektor. Der für diese Integration essentielle DNA-Bereich liegt auf dem DNA-Fragment des R-Plasmids pCxM82B, das ein Bestandteil dieses Testvektors ist.

Beispielteil

1. Konstruktion des Tn-Testvektors pK1 (Graphische Darstellung s. Abb. 2 + 3)

Der mobilisierbare E.coli-Vektor pSUP102 (Simon et al., 1983) wurde mit dem Enzym SalI linearisiert. In der gleichen Weise wurde der E.coli-Vektor pSAlE2 (E.coli-Vektor pUC19 (Norrander et al., 1983) mit dem R-Plasmid-Fragment SalE2) vorbehandelt. Die Spaltungsansätze wurden gemischt und anschließend ligiert. Mit diesem Gemisch wurde dann E.coli S17-1 (Simon et al., 2983) transformiert. Es konnte aus den Transformanten ein Plasmid isoliert werden, welches Resistenz gegen Chloramphenicol (50 μg/ml) und Erythromycon (30 μg/ml) auf Agarplatten des Antibiotic-Medium No. 3 der Firma Oxoid verleiht. Dieses Plasmid genannt pK2 besteht aus dem Vektor pSUP102 mit dem 8,1 Kb-langen R-Plasmid-Fragment SalE2.

Das Plasmid pK2 wurde mit HindIII linearisiert und anschließend religiert. Das Ligationsgemisch wurde nachE.coli S17-1 transformiert. Aus den Transformanten konnte das Plasmid pK2ΔHi (Vektor pK2 mit Deletion des HindIII-Fragments) isoliert werden. Das Plasmid pK2ΔHi wurde mit HindIII linearisiert und mit dem in gleicher Weise behandelten Vektor p2Hi4 (Plasmid PUC19 mit dem R-Plasmid-Fragment 2Hi4) gemischt und ligiert. Mit dem Ligationsgemisch wurde E.coli S17-1 transformiert. Aus den Transformanten konnte das Plasmid pK1 (Abb. 3) isoliert werden. Dieses Plasmid besteht aus einem Teil des E.coli-Vektors pSUP102 und einem ~14,4 Kb-Fragment des R-Plasmids pCxM82B und einer Gesamtgröße von ~19,7 Kb.

2. Durchführung einer Transposon-Mutagenese mit dem Plasmid pK1 zur Erzeugung von auxotrophen C. glutamicum Zellen

Der Donor E.coli S17-1/pK1 wird 20 h in 10 ml LBGEm$_{50}$ (Luria Broth mit 1 g/l Glucose (Maniatis et al., 1982) angezogen (Vorkultur).

Der Rezipient C.glutamicum ATCC 13032 wird über Nacht in 100 ml LBG bis zu einer optischen Dichte$_{580}$ =4,0 angezogen.

100 ml LBGEm$_{50}$ werden mit 1 ml der Donor-Vorkultur beimpft und bis zur optischen Dichte$_{580}$ = 1,0 angezogen.

15 ml der Rezipientenkultur werden für 9 Min. bei 49° C inkubiert.

Donor- und Rezipientenkultur werden getrennt abzentrifugiert und gewaschen.

Nach der Resuspension werden Donorzellen und Rezipienten-Zellen in einem Verhältnis von 1:1 gemischt. Das Kreuzungsgemisch (im optimalen Volumen von 0,2 ml) wird auf NC-Filter (Nitrocellulose-Filter der Firma Sartorius) getropft, die zuvor auf LBGEm $_{0,2}$-Agarplatten gelegt werden.

Das Kreuzungsgemisch wird etwa 20 h bei 37° C im Brutschrank inkubiert.

Nach der Inkubation erfolgt die Selektion des Kreuzungsgemisches auf $LBGNx_{50}Em_{25}$. Nach 2 Tagen Inkubation der Platten bei 30° C können die Erythromicin-resistenten Kolonien auf $LBGEm_{50}$. $MMEm_{50}$ (MM=Minimalmedium) Ebbinghausen et al., 1989) $LBGTc_{10}$ und $LBGCm_{10}$ übertragen werden.

Ergebnis

99 % der Integrationsmutanten sind resistent gegen Em, Tc und Cm (Resistenzgene des Vektors pK1; Em[r] und Tc[r] sind Resistenzgene des R-Plasmids pCxM82B; Cm[r] ist ein Resistenzgen des Vektors pSUP102), bei diesen Transkonjuganten ist der Vektor pK1 in das Chromosom integriert. Bei 1 % der Transkonjuganten ist nur eine Resistenz gegen Erythromycin feststellbar (Hier ist ein Teilbereich der R-Plasmid-DNA in das Chromosom integriert, auf diesem DNA-Bereich des R-Plasmids pCxM82B befindet sich das Em-Resistenzgen). Die unspezifische Integration des Vektors pK1 bzw. eines Teils des Vektors in das Chromosom von C.glutamicum konnte durch Hybridisierung chromosomaler DNA von Insertanten mit Digoxygenin-dUTP-markierter R-Plasmid-DNA nachgewiesen werden.

Die DNA-Markierung wurde mit dem "DNA Labeling and Detection Kit" von Boehringer Mannheim durchgeführt. Die Durchführung erfolgte nach den Vorschriften des Herstellers. Der DNA-Transfer erfolgte nach der Methode von Southern (1975).

Pro Kreuzungsansatz werden unter den oben aufgeführten Bedingungen etwa 130 - 150 Transkonjuganten erhalten. Auxotrophe Zellen werden mit einer Frequenz von 0,5 % bis 1 % unter diesen Transkonjuganten gefunden.

Bei den bisher durchgeführten Transposon-Mutagenese Ansätzen wurden 3 auxothophe C.glutamicum-Zellen al Leu[-], A2 Trp[-], A3 Ser[-]) isoliert. Diese Zellen wachsen nicht auf $MMEm_{50}$. Zum Feststellen der Auxotrophie wurden diese Zellen nach der Methode von Holliday (Holliday, 1956) supplementiert.

Damit hat sich gezeigt, daß der Vektor pK1 nach Mobilisierung von E.coli nach C.glutamicum unspezifisch in das Chromosom von C.glutamicum integriert. Diese unspezifische Integration des R-Plasmid-Fragments ins Chromosom wurde durch Hybridisierung der Gesamt-DNA der Insertanten mit markiert R-Plasmid-DNA nachgewiesen. Die Fähigkeit der unspezifischen Integration ist an die Anwesenheit des ~14,4 Kb R-Plasmid-Fragments gebunden und wird mit dem Vorhandensein eines Transposons bzw. eines Insertionselementes erklärt.

Abkürzungen

C.glutamicum, Corynebakterium glutamicum; cm, Chloramphenicol; E.coli, Escherichia coli; Em, Erythromycin; $Em_{25}$, 25 µg Erythromycin pro Milliliter Kulturmedium; Km, Kanamycin; Leu[-], Leucin-Auxotrophie; LBG, Luria Broth mit 1g Glucose; ug, Mikrogrann; Nx, Nalidixinsäure; $Nx_{50}$, 50 µg Nalidixinsäure pro Milliliter Kulturmedium; Nc, Nitrocellulose-Filter; pCxM82B, Plasmid aus dem Stamm Corynebakterium xerosis M82B; pK2ΔHi, Plasmid pK2 mit einer Deletion des internen HindIII-Fragments; R-Plasmid, Resistenz-Plasmid; Ser[-], Serin-Auxotrophie; Tc, Tetracyclin, Tn, Transposon, Trp[-], Tryptophan-Auxotrophie

Literatur

Berg et al., 1975, Proc.Natl.Acad.Sci., US 72, 3628ff

Ebbinghausen et al., 1989 Arch. Microbiol. 151, 238ff

Holliday, 1956 Nature 4540, 987

Kleckner et al., 1981 Ann.Rev.Genet.15, 341ff

Kuramitsu and Casadaban, 1986, J.Bact. 167, 711f

Maniatis et al., 1982 Molecular cloning, Cold Spring Harbor Lab.

Maria do Carmo de Freire Bastos and Ellen Murphy, 1988 EMBO Journal 7, 2935ff

Mc Laughlin and Hughes, 1989, J.Gen.Microbiol., 135, 2329ff

Norrander et al., 1983, Gene 26, 101ff

Perkins and Youngman, 1984, Plasmisd 12, 119ff

Simon et al, 1983, Biotechnology 1; 1983, Methods in Enzymology 118, 641-659

Simon et al, 1989, Gene 80, 161ff

Southern, 1975, J.Mol.Biol. 98, 503ff

Tomich et al., 1980 J.Bact. 141, 1366ff

Vandeyar and Zahler, 1986 J.Bact. 167, 530ff

**Patentansprüche**

1. Transposon, bestehend aus einem Fragment von ~14,4kB des R-Plasmids pCxM82B, welches enthalten ist in Corynebakterium xerosis DSM 5021, bei dem der für die Integration essentielle DNA-Bereich zwischen den Koordinaten 30,4 - 44,8 kB auf dem R-Plasmid liegt, das durch eine SalI-Schnittstelle (Koordinate 30,4 Kb) und eine HindIII-Schnittstelle (Koordinate 44,8 Kb) begrenzt wird und für eine Tetracyclin- und eine Erythromycinresistenz codierende DNA-Abschnitte aufweist.

2. Mobilisierbarer, nicht selbsttransferierbarer Vektor (Testvektor), zusammengesetzt aus:
   a) einem DNA-Segment, enthaltend ein in E.coli funktionelles Replikon,
   b) einem zweiten DNA-Segment, enthaltend für die Mobilisierungs- und Transferfunktionen codierende DNA-Fragmente (Mob-site und ori T) und
   c) das Transposon gemäß Anspruch 1.

3. Testvektor gemäß Anspruch 2,
   dadurch gekennzeichnet, daß der Vektor ein 1,9 Kb großes DNA-Fragment (Mob-site) des Plasmids RP4 trägt.

4. Testvektor gemäß Anspruch 2 oder 3,
   dadurch gekennzeichnet, daß man den E.coli Vektor aus der Gruppe pSUP101, pSUP102, pSU201, pSUP 202, pSUP203, pSUP205, pSUP301, pSUP401 auswählt.

5. Testvektor pK1 gemäß Anspruch 4,
   dadurch gekennzeichnet, daß er eine Größe von ~19,7 Kb aufweist und aus dem Transposon und einem durch eine HindIII- und eine SalI-Schnittstelle begrenzten Fragment des Vektors pSUP102 von ~ 5,3 Kb Länge besteht.

6. Verfahren zur Mutagenese eines Gram-positiven Bakteriums,
   dadurch gekennzeichnet, daß man das Transposon gemäß Anspruch 1 mit Hilfe eines Trägerplasmids durch Transformation in den Rezipienten einführt, wobei sich das Trägerplasmid dort nicht oder nur- bei niedrigen Inkubationstemperaturen repliziert.

7. Verfahren zur Mutagenes eines Gram-positiven Bakteriums durch konjugativen Transfer von mobilisierbaren Vektoren aus E.coli Mobilisatorstämmen,
   dadurch gekennzeichnet, daß man restriktionsdefekte Zellen des Gram-positiven Bakteriums herstellt und diese nach an sich bekannten Verfahren mit einem den mobilisierbaren Vektor, in dem das Transposon gemäß Anspruch 1 enthalten ist, tragenden E.coli Stamm kreuzt.

8. Verfahren gemäß Anspruch 7,
   dadurch gekennzeichnet, daß man den Testvektor pK1 gemäß Anspruch 4 verwendet.

9. Gram-positives Bakterium, insbesondere coryneformes Bakterium, transformiert durch einen das Transposon gemäß Anspruch 1 tragenden Testvektor.

10. Gram-positives Bakterium, insbesondere coryneformes Bakterium, transformiert durch den Testvektor pK1 gemäß Anspruch 4.

11. Gram-positives Bakterium gemäß Anspruch 10 mit einer durch die Transformation erzeugten Auxotrophie.

**Claims**

1. A transposon, consisting of a ~14.4 kb fragment of the R plasmid pCxM82B, which is contained in Corynebacterium xerosis DSM 5021, in which the DNA region essential for integration lies between the coordinates 30.4 and 44.8 kb on the R plasmid, and which is bounded by a SalI cleavage site (coordinate 30.4 kb) and a HindIII cleavage site (coordinate 44.8 kb) and has DNA sections coding for tetracycline and erythromycin resistance.

2. A mobilizable, non-autotransferable vector (test vector) composed of:

EP 0 445 385 B1

a) a DNA segment containing replicon functional in E. coli,

b) a second DNA segment containing DNA fragments coding for the mobilization and transfer functions (mob site and ori T), and

c) the transposon according to Claim 1.

3. A test vector according to Claim 2, characterized in that the vector carries a 1.9 kb DNA fragment (mob site) of plasmid RP4.

4. A test vector according to Claim 2 or 3, characterized in that the E. coli vector is selected from the group comprising pSUP101, pSUP102, pSU201, pSUP202, pSUP203, pSUP205, pSUP301 and pSUP401.

5. A test vector pK1 according to Claim 4, characterized in that it has a size of ~19.7 kb and consists of the transposon and a fragment of vector pSUP102 of ~5.3 kb in length, bounded by a HindIII cleavage site and a SalI cleavage site.

6. A process for the mutagenesis of a Gram-positive bacterium, characterized in that the transposon according to Claim 1 is introduced into the recipient by transformation, with the aid of a carrier plasmid, the latter not replicating in said recipient or only replicating at low incubation temperatures.

7. A process for the mutagenesis of a Gram-positive bacterium by conjugative transfer of mobilizable vectors from E. coli mobilizer strains, characterized in that restriction-defective cells of the Gram-positive bacterium are prepared and these are crossed by methods known per se with an E. coli strain carrying the mobilizable vector in which the transposon according to Claim 1 is contained.

8. A process according to Claim 7, characterized in that the test vector pK1 according to Claim 4 is used.

9. A Gram-positive bacterium, especially a corynebacterium, transformed by a test vector carrying the transposon according to Claim 1.

10. A Gram-positive bacterium, especially a corynebacterium, transformed by the test vector pK1 according to Claim 4.

11. A Gram-positive bacterium according to Claim 10 with auxotrophy produced by transformation.

## Revendications

1. Transposon, constitué d'un fragment de ~14,4 Kb du plasmide R pCxM82B, qui est compris dans corynebactérium xerosis DSM5021, dans lequel le domaine d'ADN essentiel à l'intégration est compris entre les coordonnées 30,4-44,8 Kb sur le plasmide R, qui est délimité par une zone de coupe SalI (coordonnée 30,4 Kb) et une zone de coupe HindIII (coordonnée 44,8 Kb) et qui comprend une fraction d'ADN codant pour une résistance à la tétracycline et une résistance à l'érythromycine.

2. Vecteur mobilisable, non autotransférable (vecteur test) composé de :

a) un segment d'ADN, contenant un replicon fonctionnel dans E. Coli,

b) un deuxième segment d'ADN contenant des fragments d'ADN codant pour les fonctions de mobilisation et de transfert (site Mob et oriT) et

c) le transposon selon la revendication 1.

3. Vecteur test selon la revendication 2, caractérisé en ce que le vecteur porte un fragment d'ADN de ~ 1,9 Kb du plasmide RP4 (site Mob).

4. Vecteur test selon les revendications 2 ou 3, caractérisé en ce qu'on choisit le vecteur E. Coli dans le groupe pSUP101, pSUP102, pSUP201, pSUP202, pSUP203, pSUP205, pSUP301, pSUP401.

5. Vecteur test pK1 selon la revendication 4, caractérisé en ce qu'il présente une grandeur de ~ 19,7 Kb et qu'il est constitué du transposon et d'un fragment du vecteur pSUP102, délimité par une zone de coupe HindIII et une zone SalI, d'une longueur de ~ 5,3 Kb.

6. Procédé de mutagenèse d'une bactérie gram positive, caractérisé en ce qu'on introduit le transposon se-

7

lon la revendication 1 à l'aide d'un plasmide porteur par transformation dans le receveur, le plasmide porteur ne s'y repliquant pas ou seulement à de basses températures d'incubation.

7. Procédé de mutagenèse d'une bactérie gram positive par transfert conjugué de vecteurs mobilisables de souches mobilisatrices E. Coli , caractérisé en ce qu'on crée des cellules à défaut de restriction de la bactérie gram positive et qu'on les croise dans le procédé connu avec la souche E. Coli portant le vecteur mobilisable dans lequel se trouve le transposon selon la revendication 1.

8. Procédé selon la revendication 7, caractérisé en ce qu'on utilise le vecteur test pK1.

9. Bactérie gram positive, notamment bactérie corynéforme, transformée par un vecteur test portant le transposon selon la revendication 1.

10. Bactérie gram positive, notamment bactérie corynéforme, transformée par le vecteur test pK1 selon la revendication 4.

11. Bactérie gram positive selon la revendication 10 avec une auxotrophie produite par la transformation.

<u>Abb.1</u> Restriktionskarte des R·Plasmids pCxM82B

<u>Abkürzungen</u> Ba, BamHI; Bg, BglII; Cl, ClaI; E. EcoRI; EV, EcoRV; H. HindIII; Kp, KpnI; Ns, NsiI; P, PscI; S, SalI; Xb, XbaI; Hp, HpaI

ABB.2

E,1

Xb 1400 Cl,1500
Hi,1500
Ev,1650
Ba,1850
S,2200

Cm
II

ori T

pK2
14000 bps

10300,S

9200,Xb

Em

Kp,5300
Kp,5800
Bg,6300
Ba,6400
Hi,6500

✕ Hind III
Relig.
↓
pK2ΔHi

✗ Hind III

+

ZHi4  (10600 bps)

Hi
Ba
Bg    Kp    Kp                    S              Ba        Ba    P    P         S         P    Ns  S  Hi
                                              S                P  P                   P

Em

Tc

E,1

Hi,1500
Xb,1400 Cl,1800 S,1800
Ns,2200
P,2400

Cm
I

ori T

S,3500

15900,S

P,4400
P,4600
P,4900

pK1
19600 bps

Ba,5200

14800,Xb

Tc

S,6000
Ba,6300

Em

S,7800

12100,Hi
12000,Ba
11900,Bg
11400,Kp
10900,Kp

ABB.3